# EUROPEAN PATENT SPECIFICATION

(11) **EP 4 066 892 B1**
(45) Date of publication and mention of the grant of the patent: **10.12.2025**
(21) Application number: 20892557.8
(22) Date of filing: 14.10.2020
(51) Int. Cl.: A61P 3/02, A23K 10/20, A23K 20/147, A23L 33/17, A61K 38/17

(54) **COMPOSITION FOR ENTERAL INTAKE**
ZUSAMMENSETZUNG FÜR DIE ENTERALE AUFNAHME
COMPOSITION POUR ADMINISTRATION PAR VOIE ENTÉRALE

(30) Priority: 29.11.2019 JP 2019216703
(43) Date of publication of application: 05.10.2022
(73) Proprietor: Morinaga Milk Industry Co., Ltd., Minato-ku Tokyo 105-7122 (JP)
(72) Inventor: KONO, Gaku, Zama-shi, Kanagawa 252-8583 (JP); SAKATA, Yasuyuki, Zama-shi, Kanagawa 252-8583 (JP); MORITA, Shunsuke, Zama-shi, Kanagawa 252-8583 (JP)
(74) Representative: Hoffmann Eitle
(86) International application number: PCT/JP2020/038777
(87) International publication number: WO 2021/106408

(56) References cited:
- JP-A- 2013 176 357
- JP-A- 2018 130 047
- JP-A- 2018 530 328
- KR-B1- 100 421 178
- US-A- 5 728 678
- US-A1- 2011 008 485
- US-A1- 2019 069 589
- MARTIN WILLIAM F ET AL: "Dietary protein intake and renal function", NUTRITION & METABOLISM, BIOMED CENTRAL. LONDON, GB, vol. 2, no. 1, 20 September 2005 (2005-09-20), pages 25, XP021010978, ISSN: 1743-7075, DOI: 10.1186/1743-7075-2-25

## Description

### Technical Field

The present invention relates to a composition for enteral intake.

### Background Art

From the viewpoint of nutritional physiology, protein ingestion is indispensable for animals, including humans. However, excessive ingestion of protein places a burden on the kidney and the liver. The protein intake is often controlled, especially in elderly people and people with renal or liver disease, since the burden on the kidney and the liver needs to be reduced in those people. Furthermore, in people who routinely ingest a large amount of protein, such as sport athletes, careful attention should be paid to declines in renal and liver functions.

A variety of foods with low protein content have so far been proposed for the purpose of reducing the burden on the kidney and the liver (for example, Patent Document 1).

US5728678A discloses an enteral composition for providing nutrition to a patient suffering from or at risk of renal failure, based on a mixture of amino acids.

KR100421178B1 discloses an enteral nutrition for patients with renal failure, comprising sodium caseinate.

PTL 1: JP-A-2018-130047 Hereinbelow, reference to a composition of the invention shall mean a reference to the use of said composition as defined in the claims.

### Summary of Invention

The invention pertains to the subject-matter of the claims.

### Technical Problem

Since protein is said to place a burden on the kidney and the like, it is difficult for people with decreased renal function or liver function to achieve reduction of the burden on the kidney and the liver while ingesting the required amount of protein.

In view of such circumstances, an object of the invention is to provide a protein-containing composition that can be ingested without reducing protein intake, and without placing a burden on the kidney

### Solution to Problem

In order to achieve the above object, the present inventors intensively studied to discover that micellar casein, which is a casein in a state where a micellar structure is formed, places less burden on the kidney and the liver compared to caseins having other structures and to other proteins, thereby completing the invention.

More specifically, one aspect of the invention is a protein-containing composition as defined in the claims.

The composition of the present aspect preferably comprises at least 90% by mass micellar casein with respect to the total protein.

The composition of the present aspect preferably comprises 0.1 to 25 g/100 kcal of protein.

The composition of the present aspect preferably comprises 0.01 to 3 g/100 kcal of ash.

Another aspect of the invention is a composition for enteral intake, comprising 0.1 to 25 g/100 kcal of total protein, of which at least 81% is micellar casein by mass, with respect to the total protein as further defined in the claims.

### Advantageous Effects of Invention

Since the composition of the invention places only a low burden on the kidney and the liver, the composition allows a subject (for example, a human) requiring suppression or prevention of a decline(s) in renal function to ingest protein without reducing the intake amount.

Since the protein ingested from the composition of the invention can be efficiently utilized, the composition is also useful as a protein source.

### Brief Description of Drawings

Fig. 1: Fig. 1 is a graph illustrating changes in the plasma urea level over time in humans who ingested micellar casein, sodium caseinate, or whey protein, wherein the level at the time of the ingestion is regarded as 0.
Fig. 2: Fig. 2 is a graph illustrating the glomerular filtration rates (GFRs) from 30 minutes to 2 hours after administration of micellar casein or sodium caseinate to rats.
Fig. 3: Fig. 3 is a graph illustrating changes in the plasma cystatin C level over time in rats to which micellar casein or sodium caseinate was administered, wherein the level at the time of the administration is regarded as 0.

### Description of Embodiments

The invention is described below in detail. However, the invention is not limited to the following embodiments, and may be arbitrarily modified within the scope of the claims.

The composition of the invention comprises protein, of which at least 81% is micellar casein by mass, with and is further defined in the claims respect to the total protein.

In the invention, "micellar casein" is a casein that is found in milk and maintains its micellar structure, which casein is a micelle colloid (the so-called casein micelle) formed by casein protein. It is also called "micellar casein".

Micellar casein is present in dairy products such as milk and defatted milk. Micellar casein can be concentrated by, for example, subjecting defatted milk to a membrane separation treatment using an ultrafiltration membrane or a microfiltration membrane. The milk protein concentrate obtained from the defatted milk as a result of the membrane separation treatment can be used as a micellar casein material. Since the micellar casein can be obtained by the membrane separation treatment, it is not exposed to acid precipitation and/or alkaline lysis treatment, unlike caseinates such as sodium caseinate, whose production process involves such treatment. Thus, the "micellar structure", which is said to be retained by casein in milk, is thought to be maintained in the micellar casein.

In general, micellar casein derived from bovine milk is an aggregate of caseins such as αₛ₁-casein, αₛ₂-casein, β-casein, and/or κ-casein. The micellar casein has been thought to be formed by association of small micelles called submicelles. The micellar casein characteristically contains a large amount of calcium since phosphorus and calcium are involved in the spatial structures of the caseins and in the binding portions between the casein submicelles. Thus, the micellar casein in the invention may be a micelle composed of casein, phosphorus, and calcium. The micellar casein usually has a form similar to that of the micelle colloids of casein present in milk, and is colloid particles having an average particle size of 20 to 600 nm.

The micellar casein in the invention is preferably derived from milk, more preferably derived from bovine milk.

The micellar casein in the invention is preferably produced from defatted milk (skim milk) prepared by removing lipid from raw milk by a particular method. Thus, the micellar casein is preferably a casein produced by collecting naturally occurring micellar casein (also called native micellar casein) from a raw material such as milk, rather than a casein prepared by artificial formation of a micelle. More specifically, a particular fraction of defatted milk, which fraction is obtained by removing the components having particle sizes of preferably not more than 0.1 µm from the defatted milk, can be obtained as the micellar casein. Normally, the components having particle sizes of not more than 0.1 µm contained in defatted milk are a variety of components such as whey protein and carbohydrates.

The treatment for removing the components having particle sizes of not more than 0.1 µm may be carried out by passing defatted milk through a microfiltration membrane (MF membrane) having a pore size of, for example, 0.1 to 0.2 µm. The residual component on the membrane after the treatment may contain the micellar casein. The obtained micellar casein may be subjected, when necessary, to a treatment such as concentration or spray drying. In the process of preparing the micellar casein, a treatment such as acid or alkali treatment, or addition of protease, is preferably avoided.

The casein in the thus produced raw material containing native micellar casein retains the composition and the form of the micelle colloids of casein present in normal milk, and may be preferably used for the composition of the invention. Due to the properties of the operations such as membrane treatment, the raw material may be usually obtained in a form in which whey protein as well as micellar casein are present. As long as the effect of the invention is not inhibited, such contamination is acceptable. The amount of such contaminant protein is preferably not more than 19% by mass, more preferably not more than 15% by mass, still more preferably not more than 10% by mass, still more preferably not more than 5% by mass, with respect to the total protein of the raw material containing micellar casein. The ratio of the micellar casein with respect to the total protein in such a raw material is at least 81% by mass, more preferably at least 85% by mass, still more preferably at least 88% by mass, still more preferably at least 90% by mass. The upper limit may be 100% by mass. However, from the viewpoint of availability, the ratio may be not more than 97% by mass, or not more than 95% by mass.

More specifically, in the raw material used in the composition of the invention, the amount of micellar casein with respect to the total protein may be 81 to 100% by mass, 85 to 100% by mass, 88 to 100% by mass, 90 to 100% by mass, 81 to 97% by mass, 85 to 97% by mass, 88 to 97% by mass, 90 to 97% by mass, 81 to 95% by mass, 85 to 95% by mass, 88 to 95% by mass, or 90 to 95% by mass.

As the raw material containing micellar casein in the invention, a micellar casein concentrate prepared by concentrating micellar casein by membrane separation as described above, or an isolated micellar casein product, may be used. A commercially available product may also be used. For example, a micellar casein concentrate or an isolated micellar casein product manufactured by Milei GmbH (Germany), may be used.

Casein materials have been conventionally obtained from defatted milk. However, casein materials prepared by methods such as acid precipitation and/or alkaline lysis treatment are distinguished from the effective component in the invention since those casein materials do not retain the form of micelle colloids.

For example, in conventional preparation of casein, defatted milk prepared by removal of lipid from raw milk is subjected to an acid coagulation reaction by addition of an acid, such as sulfuric acid, or to an enzymatic reaction by addition of rennet, followed by carrying out a preheating step to separate gelled protein, and a warming step for solubilization of precipitated casein using an alkali such as sodium hydroxide, to provide the casein as caseinate. A casein obtained by separation from whey by acid coagulation reaction is generally called "acid casein (material)". A casein obtained by subjecting acid casein to sodium hydroxide treatment is generally called "sodium caseinate (material)".

For the composition of the invention, a raw material containing micellar casein, obtained by such a method may be used. The blending ratio of the raw material containing micellar casein, for example, in the amount described above is preferably 1 to 15% by mass, more preferably 1 to 13% by mass, still more preferably 1 to 10% by mass, still more preferably 2 to 10% by mass.

The amount of micellar casein with respect to the total amount of protein in the composition of the invention is at least 81% by mass, preferably at least 85% by mass, more preferably at least 88% by mass, still more preferably at least 90% by mass. The upper limit of the amount of micellar casein in the composition of the invention is not limited. The amount may be not more than 100% by mass, may be not more than 97% by mass, or may be not more than 95% by mass with respect to the total protein.

When the composition of the invention contains a protein other than micellar casein, the protein is not limited as long as the effect of the invention is not inhibited. For example, the protein may be a casein protein other than micellar casein, may be a milk protein such as whey protein, may be a vegetable protein such as soybean or maize protein, or may be an animal protein such as meat or fish.

Furthermore, when appropriate, lipids, carbohydrates, minerals, vitamins, and the like may be included in the composition of the invention in addition to the protein. Examples of the mineral components may include sodium, calcium, iron, magnesium, copper, chlorine, iodine, manganese, selenium, chromium, and molybdenum. Examples of the vitamin components may include vitamin A, retinol, vitamin D, vitamin E, vitamin K, vitamin B1, vitamin B2, niacin, vitamin B6, folic acid, vitamin B12, biotin, pantothenic acid, vitamin C, and choline. Examples of the lipid components may include saturated fatty acids, n-6 fatty acids, and n-3 fatty acids. Addition of an omega-3 fatty acid such as EPA (eicosapentaenoic acid), DHA (docosahexaenoic acid), or DPA (docosapentaenoic acid) is especially preferred.

The composition may also contain a stabilizer, a gelling agent, a polysaccharide thickener, an emulsifier, or the like, when appropriate. For example, the composition may contain pectin, agar, gellan gum, carrageenan, xanthan gum, guar gum, locust bean gum, lecithin, monoglyceride, polyglyceride, or the like.

The calorie of the composition of the invention per 1 ml is preferably 0.2 to 4.0 kcal, more preferably 0.4 to 3.0 kcal, still more preferably 0.6 to 2.0 kcal.

The composition of the invention can contain protein in an amount of preferably 0.1 to 25 g/100 kcal, more preferably 0.1 to 15 g/100 kcal, still more preferably 0.1 to 10 g/100 kcal, still more preferably 2.5 to 6.0 g/100 kcal, still more preferably 3.2 to 5.5 g/100 kcal. When the composition of the invention is present in the food or beverage described herein, the amount of the protein can be measured with respect to the amount of energy in the whole food or beverage.

The composition of the invention can contain lipid in an amount of preferably 1.8 to 7.0 g/100 kcal, more preferably 2.2 to 6.5 g/100 kcal, still more preferably 2.2 to 5.4 g/100 kcal. When the composition of the invention is present in the food or beverage described herein, the lipid amount can be measured with respect to the amount of energy in the whole food or beverage.

The composition of the invention can contain carbohydrate in an amount of preferably 7.0 to 22.0 g/100 kcal, more preferably 8.5 to 21.0 g/100 kcal, still more preferably 12.0 to 17.5 g/100 kcal. When the composition of the invention is present in the food or beverage described herein, the carbohydrate amount can be measured with respect to the amount of energy in the whole food or beverage.

The composition of the invention can contain ash in an amount of preferably 0.01 to 3.0 g/100 kcal, more preferably 0.01 to 1.0 g/100 kcal, still more preferably 0.03 to 0.9 g/100 kcal, still more preferably 0.05 to 0.8 g/100 kcal.

The ash herein means sodium, calcium, iron, magnesium, zinc, and/or the like.

Although bovine milk usually contains 0.5 to 1.0 g/100 kcal of ash, the composition of the invention is distinguished from bovine milk itself.

The composition of the invention can contain sodium in an amount of preferably 30 to 250 mg/100 kcal, more preferably 43 to 240 mg/100 kcal, still more preferably 70 to 200 mg/100 kcal. When the composition of the invention is present in the food or beverage described herein, the amount of sodium can be measured with respect to the amount of energy in the whole food or beverage.

The composition of the invention can contain calcium in an amount of preferably 20 to 150 mg/100 kcal, more preferably 30 to 145 mg/100 kcal, still more preferably 50 to 120 mg/100 kcal. When the composition of the invention is present in the food or beverage described herein, the amount of calcium can be measured with respect to the amount of energy in the whole food or beverage.

The composition of the invention can contain water in an amount of preferably 30 to 100 g/100 kcal, more preferably 40 to 100 g/100 kcal, still more preferably 50 to 90 g/100 kcal. When the composition of the invention is present in the food or beverage described herein, the amount of water can be measuredwith respect to the amount of energy in the whole food or beverage.

The composition of the invention can contain potassium in an amount of preferably 25 to 240 mg/100 kcal, more preferably 30 to 225 mg/100 kcal, still more preferably 50 to 190 mg/100 kcal.

The composition of the invention can contain phosphorus in an amount of preferably 35 to 160 mg/100 kcal, more preferably 35 to 145 mg/100 kcal, still more preferably 35 to 120 mg/100 kcal.

The composition of the invention can contain vitamin A in an amount of preferably 35 to 220 µg RAE/100 kcal, more preferably 40 to 210 µg RAE/100 kcal, still more preferably 75 to 180 µg RAE/100 kcal.

The composition of the invention can contain vitamin D in an amount of preferably 0.10 to 6.0 µg/100 kcal, more preferably 0.12 to 5.7 µg/100 kcal, still more preferably 0.4 to 4.8 µg/100 kcal.

The composition of the invention can contain vitamin E in an amount of preferably 0.5 to 7.0 mg/100 kcal, more preferably 0.9 to 6.5 mg/100 kcal, still more preferably 1.0 to 5.5 mg/100 kcal.

The composition of the invention can contain vitamin K in an amount of preferably 1.2 to 11 µg/100 kcal, more preferably 1.4 to 10.5 µg/100 kcal, still more preferably 7.0 to 8.5 µg/100 kcal.

The composition of the invention can contain vitamin B1 in an amount of preferably 0.05 to 0.3 mg/100 kcal, more preferably 0.1 to 0.25 mg/100 kcal, still more preferably 0.1 to 0.16 mg/100 kcal.

The composition of the invention can contain vitamin B2 in an amount of preferably 0.10 to 0.30 mg/100 kcal, more preferably 0.13 to 0.28 mg/100 kcal, still more preferably 0.15 to 0.23 mg/100 kcal.

The composition of the invention can contain niacin in an amount of preferably 1.0 to 4.0 mg/100 kcal, more preferably 1.2 to 3.8 mg/100 kcal, still more preferably 2.0 to 3.2 mg/100 kcal.

The composition of the invention can contain vitamin B6 in an amount of preferably 0.15 to 1.3 mg/100 kcal, more preferably 0.18 to 1.2 mg/100 kcal, still more preferably 0.3 to 1.0 mg/100 kcal.

The composition of the invention can contain vitamin B12 in an amount of preferably 0.15 to 0.8 µg/100 kcal, more preferably 0.2 to 0.7 µg/100 kcal, still more preferably 0.3 to 0.6 µg/100 kcal.

The composition of the invention can contain folic acid in an amount of preferably 20 to 80 µg/100 kcal, more preferably 25 to 75 µg/100 kcal, still more preferably 30 to 63 µg/100 kcal.

The composition of the invention can contain pantothenic acid in an amount of preferably 0.4 to 1.7 mg/100 kcal, more preferably 0.5 to 1.6 mg/100 kcal, still more preferably 0.7 to 1.3 mg/100 kcal.

The composition of the invention can contain biotin in an amount of preferably 2.0 to 40 µg/100 kcal, more preferably 3.0 to 37 µg/100 kcal, still more preferably 5.0 to 31 µg/100 kcal.

The composition of the invention can contain vitamin C in an amount of preferably 4.0 to 100 mg/100 kcal, more preferably 5.0 to 50 mg/100 kcal, still more preferably 10 to 30 mg/100 kcal.

When the composition of the invention is present in the food or beverage described herein, the amount of each of potassium, phosphorus, vitamin A, vitamin D, vitamin E, vitamin K, vitamin B1, vitamin B2, niacin, vitamin B6, vitamin B12, folic acid, pantothenic acid, biotin, and vitamin C described above can be measured with respect to the amount of energy in the whole food or beverage.

Measurement of the content of each component in the composition of the invention may be carried out by a conventional method. More specifically, the lipid content may be measured by the Roese-Gottlieb method. The protein content may be measured by the combustion method. The carbohydrate may be measured by the subtraction method (the value calculated by subtracting the lipid content, protein content, ash content, and water content from 100%). The ash content may be measured by the direct ashing method, and the water content may be measured by the normal-pressure heating drying method. The calorie may be calculated by the Atwater method.

The composition of the invention is preferably in the semi-solid state or liquid state, and its viscosity is more preferably 1 to 30,000 mPa·s, 1 to 20,000 mPa·s, or 1 to 3000 mPa·s. The viscosity may be measured by using a type-B rotational viscometer (manufactured by Toki Sangyo Co., Ltd.) under the following conditions: measurement temperature, 20°C; rotor speed, 6 rpm.

The composition of the invention places only low burden on the kidney. Therefore, protein can be ingested without reducing the intake, and without placing an excessive burden on the kidney. Although soybean protein has also been conventionally known to place only low burden on the kidney, milk protein has a better nutritional composition, that is, a better amino-acid balance, than soybean protein (Stein, H. H., and J. K. Mathai. 2016. Use of the pig to determine digestible indispensable amino acid scores (DIAAS) in human foods. Proc. 5th Intl. Symp. Energy and Protein. Krakow, Poland, Sep. 12 to 15, 2016.). Therefore, micellar casein can be expected to be an excellent protein source.

Furthermore, since the amount of urea produced in the body after ingestion of the composition of the invention is small, it is assumed that the absorbed amino acids can be easily utilized without being uselessly excreted. Thus, also from the viewpoint of such an excellent protein utilization efficiency, the composition of the invention can be said to be a useful protein source.

Therefore, the composition of the invention can be suitably ingested by a subject requiring suppression or prevention of a decline(s) in renal function. The subject is usually an animal, preferably a and is defined in the claims mammal, more preferably a human.

Examples of the subject requiring suppression of a decline in renal function are subjects diagnosed with a kidney disease, and in addition, subjects whose serum creatinine level is 1.0 mg/dL or higher (male), or 0.8 mg/dL or higher (female), subjects whose serum urea nitrogen level is 22 mg/dL or higher, and subjects whose urine protein is positive.

Examples of the subject requiring prevention of a decline(s) in renal function herein include, but are not limited to, athletes, more specifically, subjects who continuously ingest at least 1.2 g/kg/day of protein.

The subject who ingests the composition of the invention may be an elderly person (who is 65 years old or older).

The application of the composition of the invention may be either a therapeutic use or a non-therapeutic use. The term "non-therapeutic" means a concept including no medical practice, that is, a concept including no treatment action on a human body by therapy.

In other words, another aspect of the invention is a method of allowing a subject requiring suppression or prevention of a decline(s) in renal function to ingest the composition for enteral intake of the invention.

In the present description, the term "allowing a subject to ingest the composition" may have the same meaning as the term "composition for use in an animal". The ingestion may be either spontaneous ingestion (free feeding) or forced ingestion. Thus, more specific examples of the ingestion include a process in which the composition of the invention is blended in a food or beverage, or in a feed, followed by supplying it to a subject to allow the subject to ingest the composition of the invention freely.

Another aspect, not pertaining to the invention, is a method of producing a protein-containing composition for enteral intake, characterized in that at least 81% by mass micellar casein is included with respect to the total protein in the composition, and that the composition is for ingestion by a subject requiring suppression or prevention of a decline(s) in renal function and/or liver function.

Disclosed herein is use of micellar casein in the production of a protein-containing composition for enteral intake, the composition comprising at least 81% by mass micellar casein with respect to the total protein, which composition is for ingestion by a subject requiring suppression or prevention of a decline(s) in renal function as further defined in the claims.

Disclosed herein is a micellar casein to be used in the production of a protein-containing composition for enteral intake, the composition comprising at least 81% by mass micellar casein with respect to the total protein, which composition is for ingestion by a subject requiring suppression or prevention of a decline(s) in renal function as further defined in the claims.

Still another aspect of the invention is a composition for enteral intake to be used for suppression or prevention of a decline(s) in renal function, the composition comprising protein, wherein at least 81% by mass micellar casein is present with respect to the total protein as further defined in the claims. The composition is preferably for ingestion by a subject requiring suppression or prevention of a decline(s) in renal function as defined in the claims function.

The timing of ingestion of the composition of the invention is not limited, and may be appropriately selected in accordance with the conditions of the subject.

The period of ingestion of the composition of the invention is not limited. The burden on the kidney and/or liver can be reduced even when the composition is continuously ingested for a long period (for example, for a period of as long as several weeks to several months).

The amount of the composition of the invention to be ingested is appropriately selected depending on conditions such as the age, the sex, and/or the state of the subject who ingests the composition.

The amount of the composition of the invention to be ingested in cases of an adult is, for example, as follows. The amount of the composition to be ingested by an adult per day is preferably 0.1 to 4 g/kg body weight/day, more preferably 0.2 to 2 g/kg body weight/day, still more preferably 0.4 to 1.2 g/kg body weight/day in terms of the amount of protein of micellar casein.

Irrespective of the amount and the period of ingestion, the composition of the invention may be ingested once daily, or divided among multiple times daily.

The composition of the invention is a composition for enteral intake. Regarding the enteral intake, the route to the intestine is not limited as long as the composition is absorbed into the body through the intestine. It may be in a form in which the composition is orally ingested, or in a form in which the composition is non-orally ingested as in the cases of gastric fistula and the like. The composition for enteral intake of the invention is preferably a nutrient composition, more preferably a liquid diet such as an enteral nutrition formula, a high-density liquid diet, a natural high-density liquid diet, an artificial high-density liquid diet, a polymeric formula, an oligomeric formula, a semi-solid nutrition formula, or an elemental diet.

The composition of the invention may be preferably in the form of a food or beverage, or a pharmaceutical, more preferably in the form of a food or beverage.

The composition may be in a form in which the composition itself of the invention is distributed as a food or beverage, or as a material of a food or beverage. The composition may also be in a form in which the composition of the invention is added to a food or beverage, or combined with another raw material of a food or beverage to form the food or beverage.

The food or beverage may be in any form, and may be, for example, a liquid, a paste, a gel-like solid, or a powder. Examples of the food or beverage include not only the above-described liquid diets (tube feeding diets), but also tablet confectionery; flour products such as bread, macaroni, spaghetti, noodles, cake mixes, frying flours, and bread crumbs; ready-to-eat foods such as instant noodles, cup noodles, retort/cooked foods, canned cooked foods, microwave-ready foods, instant soups/stews, instant miso soups/Japanese clear soups, canned soups, freeze-dried foods, and other ready-to-eat foods; agricultural products such as canned agricultural products, canned fruits, jellies/marmalades, pickles, boiled beans, dried agricultural products, and cereals (processed cereal products); processed fishery products such as canned fishery products, fish ham/sausage, fish-paste products, marine delicacies, and tsukudani; processed livestock products such as canned livestock products, livestock pastes, and livestock ham/sausage; milks and dairy products such as processed milks, milk beverages, yogurts, lactic acid bacteria beverages, cheeses, ice creams, powdered milk formulae, creams, and other dairy products; oils and fats such as butter, margarine, and vegetable oil; basic seasonings such as soy sauce, soybean paste (miso), sauces, processed tomato seasonings, sweet sake (mirin), and vinegar; complex seasonings and foods such as cooking mixtures, curry seasonings, bastes, dressings, noodle soup bases, spices, and other complex seasonings; frozen foods such as frozen food materials, frozen semi-cooked foods, and frozen cooked foods; confectionery such as caramels, candies, chewing gums, chocolates, cookies, biscuits, cakes, pies, snacks, crackers, Japanese confectionery, rice confectionery, bean confectionery, dessert confectionery, jellies, and other kinds of confectionery; luxury beverages such as carbonated beverages, natural fruit juices, fruit juice beverages, soft drinks containing fruit juice, nectar beverages, fruit juice beverages containing fruit pulp, vegetable-based beverages, soybean milk, soybean milk beverages, coffee beverages, tea beverages, powdered beverages, concentrated beverages, sports beverages, nutritional beverages, alcoholic beverages, and other luxury beverages; other commercially available foods such as baby foods, seasoned powders (furikake), ochazukenori, and others; powdered milk formulae for infants; enteral nutrition diets; and functional foods (foods for specified health uses and foods with nutrient function claims).

One form of the food or beverage may be a feed. Examples of the feed include pet foods, livestock feeds, and fish farming feeds.

The form of the feed is not limited, and the feed may contain a cereal such as maize, wheat, barley, rye, or milo; a vegetable oil cake such as soybean oil cake, rapeseed oil cake, palm oil cake, or linseed oil cake; a bran such as bran (fusuma), wheat bran, rice bran, or defatted rice bran; a manufactured dreg such as corn gluten meal or corn germ meal; an animal-based feed such as fish meal, non-fat dry milk, whey, yellow grease, or tallow; a yeast such as torula yeast or beer yeast; a mineral-based feed such as tricalcium phosphate or calcium carbonate; an oil or fat; a simple amino acid; a saccharide; or the like.

When the composition of the invention is in the form of a food or beverage, or is included in a food or beverage, the amount of the micellar casein and/or its hydrolysate is not limited, and may be appropriately designed based on the amount of protein to be ingested per day, and/or on the above-described amount of micellar casein to be ingested per day.

### Examples

The invention is described below more concretely by way of Examples. However, the invention is not limited to these Examples.

### <Example 1>

### (1) Test of Administration to Humans

Two subjects were instructed to orally ingest one of the samples shown in Table 1. Blood was collected from the subjects 0, 30, 60, 120, 180, and 240 minutes after the ingestion, and plasma urea was measured. The test was carried out such that different samples were ingested on different days. In the micellar casein concentrate used, the ratio of micellar casein in the total protein was 90%. The sodium caseinate and the whey protein concentrate were samples free of micellar casein.

**[Table 1]**

| (g) | | Micellar casein group | Sodium caseinate group | Whey protein group |
|---|---|---|---|---|
| Micellar casein concentrate (MC80, manufactured by Milei GmbH) | Protein: 82.4% by mass | 24.3 | - | - |
| Sodium caseinate (TATUA-100, manufactured by TATUA) | Protein: 93.5% by mass | - | 21.4 | - |
| Whey protein concentrate (Milei 80, manufactured by Milei GmbH) | Protein: 82% by mass | - | - | 24.4 |
| Maltodextrin (Starch degradation product, manufactured by Matsutani Chemical Industry Co., Ltd.) | Brix: 67% | 25.8 | 29.45 | 23.94 |
| Water | | 299.9 | 299.2 | 301.7 |
| Total | | 350 | 350 | 350 |
| Energy (kcal) | | 160 | 160 | 160 |

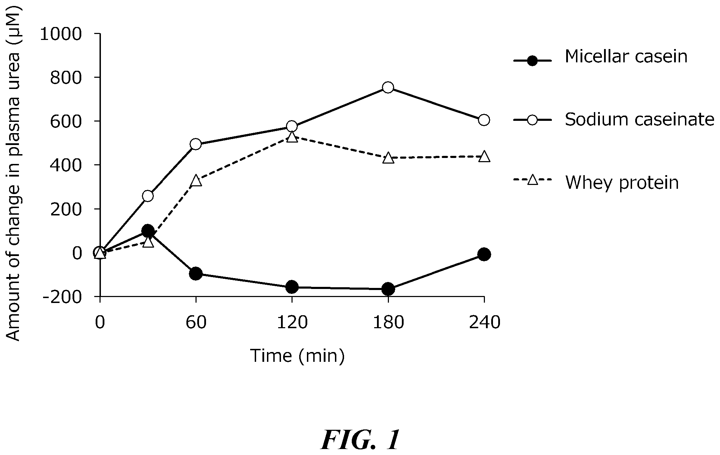

Fig. 1 illustrates the changes in the plasma urea level over time relative to the level at the time of ingestion, which is regarded as 0.

In the group in which micellar casein was ingested, the plasma urea level did not increase. It should be noted that the ingested products contained very little urea, and hence the urea was a product of biosynthesis (in the urea cycle). These results indicate that, in spite of the fact that excessive ingestion of protein is generally problematic because of the burden on the liver and the kidney, micellar casein is a protein that places only low burden on the liver and kidney.

### <Example 2>

### (2) Test of Administration to Rats

A micellar casein concentrate (MC80, manufactured by Milei GmbH), sodium caseinate (TATUA 100, manufactured by TATUA), or water was orally administered to rats (n=4 in each group). The amount of protein ingested in the protein ingestion groups was set to 1.5 g per 1 kg body weight. The glomerular filtration rate (GFR) was measured from 30 minutes to 2 hours after the administration. In addition, blood was collected 0, 30, 60, 90, 120, and 150 minutes after the administration, and the plasma cystatin C level was measured.

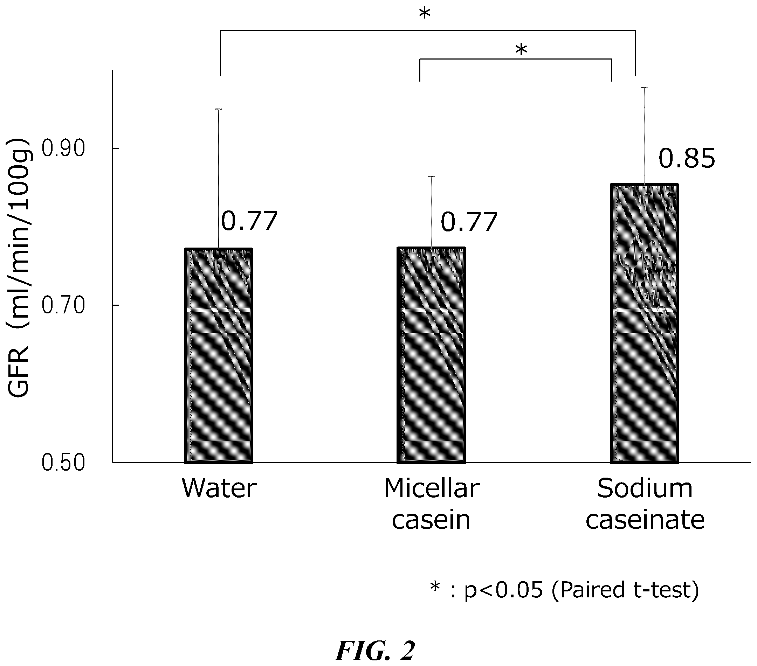

Fig. 2 illustrates the GFRs from 30 minutes to 2 hours after the administration.

In the group in which sodium caseinate was administered, the GFR increased to 0.85 mL/minute/100 g. In contrast, in the group in which micellar casein was administered, the GFR was 0.77 mL/minute/100 g, which was almost the same as in the water administration group. Since an increase in the GFR indicates a heavy burden on the kidney, it can be seen that micellar casein is a protein that places less burden on the kidney compared to sodium caseinate.

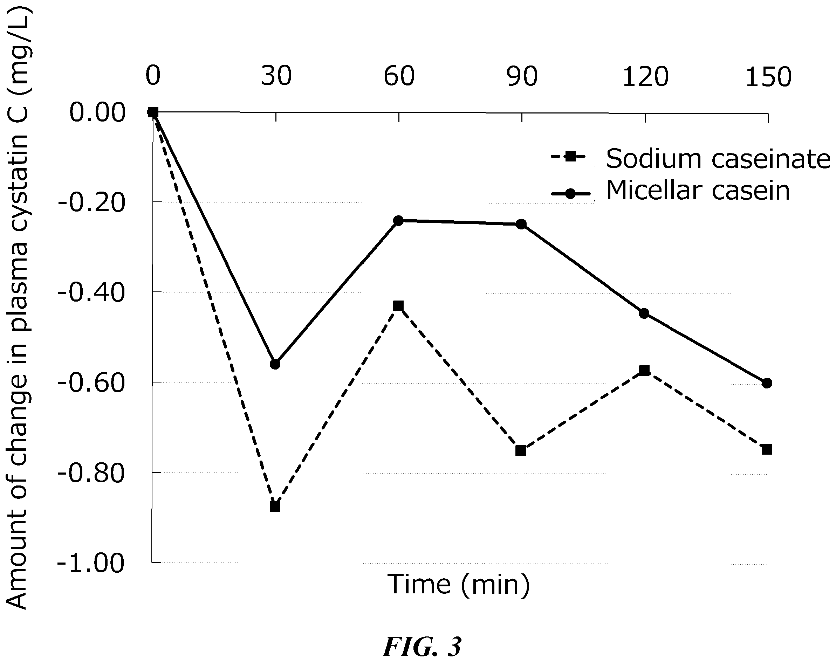

Fig. 3 illustrates the changes in the plasma cystatin C level over time.

Cystatin C is produced by cells throughout the body, and easily filtered through kidney glomeruli since it does not bind to plasma protein. Since the filtration is followed by reabsorption in the proximal renal tubule and then degradation into amino acids, the plasma cystatin C level decreases as the GFR increases. The group in which sodium caseinate was administered showed a greater decrease in cystatin C than the group in which micellar casein was administered. This result is consistent with the result on the GFR (Fig. 2), and hence it can be seen that micellar casein is a protein that places less burden on the kidney compared to sodium caseinate.

### <Production Example 1>

In 75 kg of dissolving water, 5.1 kg of a micellar casein concentrate (manufactured by Milei GmbH), 19.4 kg of maltodextrin (manufactured by Matsutani Chemical Industry Co., Ltd.), and 0.5 kg of Fiber Sol 2 (manufactured by Matsutani Chemical Industry Co., Ltd.) were dispersed and dissolved. The resulting solution was mixed with 620 g of a mineral mixture prepared according to the composition shown in Table 2. The resulting solution was mixed with 2.8 kg of soybean oil (manufactured by Taiyo Yushi Corp.) in which 50 g of succinic acid monoglyceride (manufactured by Kao Corporation) and 50 g of lecithin (manufactured by J-Oil Mills, Inc.) were dissolved as emulsifiers. The resulting mixture was pre-emulsified using a TK Homomixer (manufactured by PRIMIX Corporation), and water was added thereto to a final volume of 100 L. Subsequently, the pre-emulsified product was homogenized using a high-pressure homogenizer (manufactured by SPX Corporation) at a pressure of 50 MPa. Thereafter, the resulting emulsion was dispensed in 200-mL volumes into retort pouches (manufactured by Toyo Seikan Co., Ltd.), and the pouches were sealed. Sterilization treatment was carried out at 125°C for 15 minutes using a retort sterilizer (manufactured by Hisaka Works, Ltd.), to produce a liquid nutrient composition.

The resulting composition is suitable for suppression or prevention of a decline(s) in renal function and/or liver function, has the composition according to Table 3, and has a calorie of 100 kcal per 100 mL.

**[Table 2]**

| Raw material name | Amount added (g) |
|---|---|
| Trisodium citrate | 300 |
| Potassium carbonate | 50 |
| Potassium chloride | 170 |
| Magnesium chloride | 100 |

**[Table 3]**

| | Amount added (/100 mL) |
|---|---|
| Amount of energy | 100 kcal |
| Protein content | 4.0 g |
| Lipid content | 3.0 g |
| Carbohydrate content | 14.7 g (including 0.4 g of dietary fiber) |
| Sodium content | 76 mg |
| Potassium content | 95 mg |
| Calcium content | 118 mg |
| Magnesium content | 25 mg |
| Chlorine content | 120 mg |
| Phosphorus content | 76 mg |

## Claims

1. A protein-containing composition for use in the suppression or prevention of a decline in renal function in a subject diagnosed with kidney disease, male subjects whose serum creatinine level is 1.0 mg/dL or higher, female subjects whose serum creatinine level is 0.8 mg/dL or higher, subjects whose serum urea nitrogen level is 22 mg/dL or higher, or subjects whose urine protein is positive,
the composition comprising at least 81% by mass micellar casein with respect to the total protein.

2. The composition for the use according to claim 1, comprising at least 90% by **mass** micellar casein with respect to the total protein.

3. The composition for the use according to any one of claims 1 or2, comprising 0.1 to 25 g/100 kcal of protein.

4. The composition for the use according to any one of claims 1 - 3 comprising 0.01 to 3 g/100 kcal of ash.

5. The composition or the composition for the use according to any of the preceding claims, wherein the micellar casein is derived from milk, preferably from bovine milk and is preferably an aggregate of caseins such as αₛ₁-casein, αₛ₂-casein, β-casein, and/or κ-casein.

6. A non-therapeutic use of a protein-containing composition for enteral intake,
wherein the composition comprises at least 81% by mass micellar casein with respect to the total protein,
for prevention of a decline(s) in renal function in a subject requiring said prevention,
wherein the subject requiring prevention of a decline(s) in renal function is an athlete who continuously ingests at least 1.2 g/kg/day of protein, or a person of 65years or older.

7. The use according to claim 6, wherein the composition is further characterized as defined in any one of claims 2 -4.

## Patentansprüche

1. Protein-enthaltende Zusammensetzung zur Verwendung bei der Unterdrückung oder Verbeugung der Verschlechterung der Nierenfunktion bei einem Subjekt, bei dem eine Nierenerkrankung diagnostiziert wurde, bei männlichen Subjekten, deren Serumkreatinin-Spiegel 1,0 mg/dL oder höher ist, bei weiblichen Subjekten, deren Serumkreatinin-Spiegel 0,8 mg/dL oder höher ist, bei Subjekten, deren Serumharnstoffstickstoff-Spiegel 22 mg/dL oder höher ist, oder bei Subjekten, deren Urinprotein positiv ist, wobei die Zusammensetzung mindestens 81% Massenanteil mizellares Casein, bezogen auf das Gesamtprotein, umfasst.

2. Zusammensetzung zur Verwendung gemäß Anspruch 1, die mindestens 90% Massenanteil mizellares Casein, bezogen auf das Gesamtprotein, umfasst.

3. Zusammensetzung zur Verwendung gemäß irgendeinem der Ansprüche 1 oder 2, umfassend 0,1 bis 25 g/100 kcal Protein.

4. Zusammensetzung zur Verwendung gemäß irgendeinem der Ansprüche 1 - 3, umfassend 0,01 bis 3 g/100 kcal Asche.

5. Zusammensetzung oder die Zusammensetzung zur Verwendung nach irgendeinem der vorhergehenden Ansprüche, wobei das mizellare Casein aus Milch, vorzugsweise aus Rindermilch, gewonnen wird und vorzugsweise ein Aggregat von Caseinen wie αₛ₁-Casein, αₛ₂-Casein, β-Casein und/oder κ-Casein ist.

6. Nicht-therapeutische Verwendung einer Proteinenthaltenden Zusammensetzung zur enteralen Aufnahme, wobei die Zusammensetzung mindestens 81% Massenanteil mizellares Casein in Bezug auf das Gesamtprotein umfasst, zur Vorbeugung einer Verschlechterung oder Verschlechterungen der Nierenfunktion bei einem Subjekt, das diese Vorbeugung(en) benötigt, wobei das Subjekt, das eine Vorbeugung einer Verschlechterung oder Verschlechterungen der Nierenfunktion benötigt, ein Sportler ist, der kontinuierlich mindestens 1,2 g/kg/Tag an Protein aufnimmt, oder eine Person von 65 Jahren oder älter ist.

7. Verwendung nach Anspruch 6, wobei die Zusammensetzung ferner charakterisiert ist, wie definiert in irgendeinem der Ansprüche 2-4.

## Revendications

1. Composition contenant des protéines, destinée à une utilisation pour la suppression ou la prévention d'un déclin de la fonction rénale chez un sujet diagnostiqué insuffisant rénal, chez les sujets masculins dont le niveau de créatinine sérique est supérieur ou égal à 1,0 mg/dL, chez les sujets féminins dont le niveau de créatinine sérique est supérieur ou égal à 0,8 mg/dL, chez les sujets dont le niveau d'azote uréique sérique est supérieur ou égal à 22 mg/dL, ou chez les sujets dont la protéinurie est positive,
la composition comprenant au moins 81 % en masse de caséine micellaire caséine par rapport aux protéines totales.

2. Composition destinée à l'utilisation selon la revendication 1, comprenant au moins 90 % en **masse** de caséine micellaire par rapport aux protéines totales.

3. Composition destinée à l'utilisation selon l'une quelconque des revendications 1 ou 2, comprenant 0,1 à 25 g/100 kcal de protéines.

4. Composition destinée à l'utilisation selon l'une quelconque des revendications 1 à 3, comprenant 0,01 à 3 g/100 kcal de cendres.

5. Composition ou composition destinée à l'utilisation selon l'une quelconque des revendications précédentes, dans laquelle la caséine micellaire est dérivée du lait, de préférence du lait de vache, et est de préférence un agrégat de caséines telles que la caséine αₛ₁, la caséine αₛ₂, la caséine β, et/ou la caséine κ.

6. Utilisation non-thérapeutique d'une composition contenant des protéines pour une administration par voie entérale,
dans laquelle la composition comprend au moins 81 % en masse de caséine micellaire par rapport aux protéines totales,
pour la prévention d'un (des) déclin(s) de la fonction rénale chez un sujet nécessitant ladite prévention,
dans laquelle le sujet nécessitant la prévention d'un (des) déclin(s) de la fonction rénale est un athlète qui ingère continuellement au moins 1,2 g/kg/jour de protéines ou une personne de 65 ans ou plus.

7. Utilisation selon la revendication 6, dans laquelle la composition est en outre caractérisée selon l'une quelconque des revendications 2 à 4.
